# EUROPEAN PATENT APPLICATION

(11) **EP 4 834 317 A1**
(43) Date of publication of application: **04.11.2026**
(21) Application number: 24913052.7
(22) Date of filing: 26.12.2024
(51) Int. Cl.: A61K 8/44, A61K 31/197, A61P 25/02

(54) **AGENT FOR ALLEVIATING HYPERSENSITIVITY**

(30) Priority: 28.12.2023 JP 2023222697
(71) Applicant: Kao Corporation, Tokyo 103-8210 (JP)
(72) Inventor: SUMIDA, Yasuteru, Tokyo 103-8210 (JP)
(74) Representative: Hoffmann Eitle
(86) International application number: PCT/JP2024/046269
(87) International publication number: WO 2025/143166

(57) **Abstract**

The present invention relates to an agent for alleviating skin hypersensitivity which alleviates or relieves the degree of hypersensitivity for sensitive skin of hypersensitivity type with an apparently healthy appearance, and specifically relates to an agent for alleviating skin hypersensitivity comprising γ-amino-β-hydroxybutyric acid (A) as an active ingredient.

## Description

### Field of the Invention

The present invention relates to an agent for alleviating hypersensitivity.

### Background of the Invention

In recent years, the term "sensitive skin" has come into general use, and has been frequently featured in women's magazines and the like. When people who are aware that they have sensitive skin are asked about their symptoms, examples of specific symptoms reported include, for example, "an itchy sensation upon application of cosmetics," "a tingling feeling," "being prone to acne or skin eruptions," and "easily becoming red in response to weak stimuli."

When people who are aware that they have sensitive skin are analyzed, they can be classified into three types, for example, Type I: type with an epidermal barrier disruption, Type II: type with inflammation, and Type III: hypersensitivity type apparently having healthy skin.

For these three types, it has been considered that an appropriate care for each type is necessary in order to improve the sensitive skin to restore healthy skin. The reason for this consideration is that the causes of the sensitive skin differ depending on the type.

Among the three types, the Type I has been found to show an improvement in the barrier function and enhancement of the tolerance to stimulation by using niacinamide or the like, which has an effect to activate epidermal lipid synthesis. However, for the Type II and the Type III, no improvement has been observed by that method, because the causes of the sensitive skin differ from those of the Type I. For the Type II and the Type III, for example, past preliminary experiments have shown that the hypersensitivity tends to be alleviated by a treatment with an ingredient derived from a plant, which has an NGF (nerve growth factor) inhibitory effect, but there has been still room for further study (Reference Literature 1).

On the other hand, γ-amino-β-hydroxybutyric acid is known to be used by being incorporated into cosmetics and the like, and to have a skin anti-aging effect and a skin whitening effect (for example, Reference Literatures 2 to 4).

(Reference Literature 1) Tomohiro Yokota, "Classification of Sensitive Skin with Analysis of Skin Physiological Parameter," Journal of Japanese Cosmetic Science Society, Vol. 29, No. 1, pp. 44-49 (2005)
(Reference Literature 2) JP 1987-255405 A
(Reference Literature 3) JP 1988-10708 A
(Reference Literature 4) JP 2003-246709 A

### Summary of the Invention

The present invention relates to an agent for alleviating skin hypersensitivity that contains γ-amino-β-hydroxybutyric acid (A) as an active ingredient.

The present invention also relates to use of γ-amino-β-hydroxybutyric acid (A) as an agent for alleviating skin hypersensitivity.

The present invention also relates to a method of using γ-amino-β-hydroxybutyric acid (A) as an agent for alleviating skin hypersensitivity.

### Brief Description of the Drawing

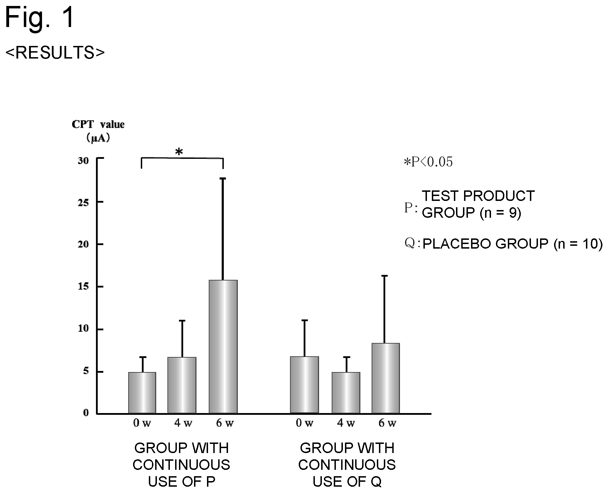

Fig. 1 is a diagram illustrating electrical stimulation thresholds (CPT values) before the start of continuous use of each lotion in Example 2, after four weeks of continuous use thereof, and after six weeks of continuous use thereof.

### Detailed Description of the Invention

Among the types of sensitive skin, in particular for the sensitive skin of hypersensitivity type with an apparently healthy appearance, there has been a demand for a topical agent which alleviates or relieves the degree of the hypersensitivity.

The present inventors found that γ-amino-β-hydroxybutyric acid has an effect to alleviate or relieve the degree of the hypersensitivity for the sensitive skin of hypersensitivity type with an apparently healthy appearance, and that the γ-amino-β-hydroxybutyric acid is useful as an agent for alleviating the skin hypersensitivity.

One aspect of the present invention is an agent for alleviating skin hypersensitivity that contains γ-amino-β-hydroxybutyric acid (A) as an active ingredient.

Further, another aspect of the present invention is use of γ-amino-β-hydroxybutyric acid (A) as an agent for alleviating the skin hypersensitivity.

Moreover, another aspect of the present invention is a method of using γ-amino-β-hydroxybutyric acid (A) as an agent for alleviating the skin hypersensitivity.

The agent for alleviating the hypersensitivity according to the present invention is capable of alleviating or relieving the degree of the hypersensitivity for the sensitive skin of hypersensitivity type with an apparently healthy appearance.

The terms used in the present specification are used in their commonly understood meanings in the art unless otherwise specified.

In the present specification, the term "skin hypersensitivity" refers to sensitive skin as described above, that is, skin which does not exhibit clear inflammation but produces sensations such as tingling, stinging, or prickling. The agent for alleviating the skin hypersensitivity according to the present invention is effective for such sensitive skin in general, and is particularly effective for the sensitive skin of hypersensitivity type with an apparently healthy appearance (the Type III described above).

Further, the term "agent for alleviating skin hypersensitivity" means an agent that alleviates or relieves the degree of the hypersensitivity.

The ingredient (A), γ-amino-β-hydroxybutyric acid, is a known substance and has the following chemical properties.
- Chemical name: γ-amino-β-hydroxybutyric acid
- Molecular formula: C₄H₉NO₃
- Molecular weight: 119.12
- Melting point: from 205 to 210°C
-

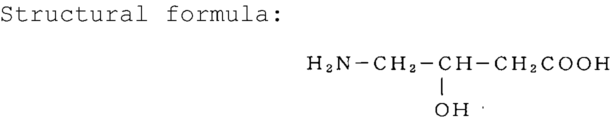

From the viewpoints of alleviating or relieving the degree of the hypersensitivity for the sensitive skin of hypersensitivity type with an apparently healthy appearance and reducing stickiness during application of the agent, the content of the ingredient (A) is preferably 0.005% by mass or more in the total composition, more preferably 0.05% by mass or more, and still more preferably 0.3% by mass or more, and is preferably 2% by mass or less, more preferably 1.5% by mass or less, and still more preferably 1% by mass or less.

In addition, the content of the ingredient (A) is preferably 0.005% by mass or more and 2% by mass or less in the total composition, more preferably 0.05% by mass or more and 1.5% by mass or less, and still more preferably 0.3% by mass or more and 1% by mass or less.

The agent for alleviating the hypersensitivity according to the present invention may further contain an ingredient (B), a polyhydric alcohol. The agent is capable of alleviating or relieving the degree of the hypersensitivity for the sensitive skin of hypersensitivity type with an apparently healthy appearance, and reducing the stickiness after the application of the agent. It should be noted that the ingredient (B) does not contain ingredient (D), an osmolyte described later in this specification.

The polyhydric alcohol is a compound having two or more hydroxyl groups in the molecule, and any such compound may be used as long as it is commonly applicable for a topical skin preparation.

Examples of dihydric alcohols include, for example, polyethylene glycol, propylene glycol, dipropylene glycol, tripropylene glycol, polypropylene glycol, 1,3-butylene glycol, propanediol, and the like. Examples of trihydric alcohols include glycerin, trimethylolpropane, and the like. Examples of tetrahydric alcohols include diglycerin, erythritol, and the like. Examples of polyhydric alcohols having five or more hydroxyl groups include polyglycerins such as triglycerin; and sugars and sugar alcohols such as glucose, maltose, maltitol, sucrose, xylitol, sorbitol, malbitol, polyoxyethylene methyl glucoside, polyoxyethylene ethyl glucoside, and polyoxyethylene propylene glucoside.

From the viewpoints of alleviating or relieving the degree of the hypersensitivity for the sensitive skin of hypersensitivity type with an apparently healthy appearance, and reducing the stickiness during the application of the agent, the polyhydric alcohol is preferably one or more selected from the group consisting of dihydric alcohols, trihydric alcohols, tetrahydric alcohols, and sugar alcohols, more preferably one or more selected from the group consisting of dihydric alcohols, trihydric alcohols, and tetrahydric alcohols, still more preferably one or more selected from the group consisting of trihydric alcohols and tetrahydric alcohols, and even more preferably one or more selected from the group consisting of glycerin and diglycerin.

The polyhydric alcohol of the ingredient (B) may be used alone or in combination of two or more. From the viewpoints of alleviating or relieving the degree of the hypersensitivity for the sensitive skin of hypersensitivity type with an apparently healthy appearance, reducing the stickiness during the application of the agent, and improving moisturizing feel, the content of the polyhydric alcohol is preferably 1% by mass or more in the total composition, more preferably 3% by mass or more, and still more preferably 5% by mass or more, and is preferably 20% by mass or less, more preferably 15% by mass or less, and still more preferably 12% by mass or less.

In addition, the content of the ingredient (B) is preferably 1% by mass or more and 20% by mass or less in the total composition, more preferably 3% by mass or more and 15% by mass or less, and still more preferably 5% by mass or more and 12% by mass or less.

The agent for alleviating the hypersensitivity according to the present invention may further contain an ingredient (C), a pH adjusting agent. The agent is capable of alleviating or relieving the degree of the hypersensitivity for the sensitive skin of hypersensitivity type with an apparently healthy appearance, and improving the stability of the preparation. It should be noted that the ingredient (C) does not contain ingredient (D), an osmolyte described later in this specification.

Any pH adjusting agent may be used as long as it is commonly applicable for a topical skin preparation, and inorganic acids and salts thereof, organic acid having six or less carbon atoms and salts thereof are preferred, and organic acids having six or less carbon atoms and salts thereof are more preferred.

The inorganic acid is preferably one or more selected from the group consisting of, for example, hydrochloric acid, nitric acid, nitrous acid, sulfuric acid, sulfurous acid, phosphoric acid, phosphonic acid, phosphinic acid, and salts thereof, and is more preferably one or more selected from the group consisting of phosphoric acid and salts thereof.

As the organic acids having six or less carbon atoms and salts thereof, water-soluble organic acids and salts thereof are preferred, and examples thereof include, for example, fatty acids having from one to six carbon atoms, hydroxy acids, dicarboxylic acids, and salts thereof. Among these, it is preferable to include one or more selected from the group consisting of malic acid, lactic acid, citric acid, succinic acid, acetic acid, tartaric acid, aspartic acid, adipic acid, and salts thereof, more preferably one or more selected from the group consisting of malic acid, lactic acid, citric acid, succinic acid, and salts thereof, and still more preferably one or more selected from the group consisting of citric acid and salts thereof.

The pH adjusting agent of the ingredient (C) may be used alone or in combination of two or more. From the viewpoints of alleviating or relieving the degree of the hypersensitivity for the sensitive skin of hypersensitivity type with an apparently healthy appearance, improving the stability of the preparation, and providing pH buffering capacity, the content of the ingredient (C) is preferably 0.01% by mass or more in the total composition, more preferably 0.03% by mass or more, and still more preferably 0.05% by mass or more, and is preferably 1% by mass or less, more preferably 0.5% by mass or less, and still more preferably 0.3% by mass or less.

In addition, the content of the ingredient (C) is preferably 0.01% by mass or more and 1% by mass or less in the total composition, more preferably 0.03% by mass or more and 0.5% by mass or less, and still more preferably 0.05% by mass or more and 0.3% by mass or less.

The agent for alleviating the hypersensitivity according to the present invention may further contain an ingredient (D), an osmolyte. The agent is capable of alleviating or relieving the degree of the hypersensitivity for the sensitive skin of hypersensitivity type with an apparently healthy appearance, and reducing the stickiness after the application of the agent. It should be noted that the ingredient (D) does not contain the ingredient (B), a polyhydric alcohol, nor the ingredient (C), a pH adjusting agent.

Examples of the osmolyte include, for example, ectoine, sodium pyrrolidone carboxylate (PCA-Na), arginine, arginine hydrochloride, carnitine chloride, taurine, glutamic acid, glutamate, serine, N-methyl-L-serine, trimethylglycine, alanine, and the like.

From the viewpoints of alleviating or relieving the degree of the hypersensitivity for the sensitive skin of hypersensitivity type with an apparently healthy appearance, and reducing the stickiness during the application of the agent, the osmolyte is preferably one or more selected from the group consisting of ectoine, sodium pyrrolidone carboxylate (PCA-Na), arginine, N-methyl-L-serine, and alanine, more preferably one or more selected from the group consisting of ectoine, sodium pyrrolidone carboxylate (PCA-Na), and N-methyl-L-serine, and is still more preferably sodium pyrrolidone carboxylate (PCA-Na).

The osmolyte of the ingredient (D) may be used alone or in combination with two or more additional osmolytes. From the viewpoints of alleviating or relieving the degree of the hypersensitivity for the sensitive skin of hypersensitivity type with an apparently healthy appearance, and reducing the stickiness during the application of the agent, the content of the ingredient (D) is preferably 0.015% by mass or more in the total composition, more preferably 0.02% by mass or more, and still more preferably 0.5% by mass or more, and is preferably 3.5% by mass or less, more preferably 3% by mass or less, and still more preferably 2% by mass or less.

In addition, the content of the ingredient (D) is preferably 0.015% by mass or more and 3.5% by mass or less in the total composition, more preferably 0.02% by mass or more and 3% by mass or less, and still more preferably 0.5% by mass or more and 2% by mass or less.

In the present invention, from the viewpoints of uniformly dissolving the ingredients (A) and (D) and reducing the stickiness during the application of the agent, the content of water is preferably 20% by mass or more in the total composition, more preferably 40% by mass or more, and still more preferably 60% by mass or more, and is preferably 98.975% by mass or less, more preferably 90% by mass or less, and still more preferably 85% by mass or less.

In addition, the content of water is preferably 20% by mass or more and 98.975% by mass or less in the total composition, more preferably 40% by mass or more and 90% by mass or less, and still more preferably 60% by mass or more and 85% by mass or less.

In addition to the ingredients described above, the topical skin preparation of the present invention may contain ingredients commonly applicable to a topical skin preparation, such as oil-based ingredients, surfactants, preservative agents, antioxidant agents, colorants, fragrances, plant extracts, coloring agents, powders, ultraviolet absorbing agents, moisturizing agents other than the ingredient (B), blood circulation promoting agents, cooling agents, antiperspirant agents, bactericides, and skin activating agents.

The agent for alleviating the hypersensitivity according to the present invention can be produced according to an ordinary method.

For example, it can be produced by mixing and stirring all ingredients including the ingredient (A).

From the viewpoint of enhancing transdermal absorbability, the agent for alleviating the hypersensitivity according to the present invention preferably has a pH value of from 4 to 7, more preferably from 4.5 to 6.5, and still more preferably from 5 to 6.

In the present invention, the pH value is measured at 25°C using a bench-top pH meter F-72 manufactured by HORIBA, Ltd.

The agent for alleviating the hypersensitivity according to the present invention can be applied to a topical skin preparation, a cosmetic, a quasi-drug medicated cosmetic, a pharmaceutical, and the like, and is suitable as a cosmetic, more suitable as a cosmetic for skin care.

Regarding the embodiments described above, the present invention further discloses the following compositions.
<1> An agent for alleviating skin hypersensitivity, comprising γ-amino-β-hydroxybutyric acid (A) as an active ingredient.
<2> The agent for alleviating the hypersensitivity according to <1>, wherein the content of the ingredient (A) is preferably 0.005% by mass or more and 2% by mass or less in the total composition, more preferably 0.05% by mass or more and 1.5% by mass or less, and still more preferably 0.3% by mass or more and 1% by mass or less.
<3> The agent for alleviating the hypersensitivity according to <1> or <2>, further comprising a polyhydric alcohol (B).
<4> The agent for alleviating the hypersensitivity according to <3>, wherein the polyhydric alcohol (B) preferably is one or more selected from the group consisting of dihydric alcohols, trihydric alcohols, and tetrahydric alcohols, more preferably is one or more selected from the group consisting of trihydric alcohols and tetrahydric alcohols, and still more preferably is one or more selected from the group consisting of glycerin and diglycerin.
<5> The agent for alleviating the hypersensitivity according to <3> or <4>, wherein the content of the polyhydric alcohol (B) is preferably 1% by mass or more and 20% by mass or less in the total composition, more preferably 3% by mass or more and 15% by mass or less, and still more preferably 5% by mass or more and 12% by mass or less.
<6> The agent for alleviating the hypersensitivity according to any one of <1> to <5>, further comprising a pH adjusting agent (C).
<7> The agent for alleviating the hypersensitivity according to any one of <1> to <6>, wherein the agent has a pH value of from 4 to 7.
<8> The agent for alleviating the hypersensitivity according to any one of <1> to <7>, further comprising an osmolyte (D).
<9> The agent for alleviating the hypersensitivity according to <8>, wherein the osmolyte (D) preferably contains one or more selected from the group consisting of ectoine, sodium pyrrolidone carboxylate (PCA-Na), arginine, N-methyl-L-serine, and alanine, more preferably contains one or more selected from the group consisting of ectoine, sodium pyrrolidone carboxylate (PCA-Na), and N-methyl-L-serine, and still more preferably contains sodium pyrrolidone carboxylate (PCA-Na).
<10> The agent for alleviating the hypersensitivity according to <8> or <9>, wherein the content of the osmolyte (D) is preferably 0.015% by mass or more and 3.5% by mass or less in the total composition, more preferably 0.02% by mass or more and 3% by mass or less, and still more preferably 0.5% by mass or more and 2% by mass or less.
<11> The agent for alleviating the hypersensitivity according to any one of <1> to <10>, further comprising water, wherein the content of water is preferably 20% by mass or more and 98.975% by mass or less in the total composition, more preferably 40% by mass or more and 90% by mass or less, and still more preferably 60% by mass or more and 85% by mass or less.
<12> Use of γ-amino-β-hydroxybutyric acid (A) as an agent for alleviating skin hypersensitivity.
<13> A method of using γ-amino-β-hydroxybutyric acid (A) as an agent for alleviating skin hypersensitivity.

### Examples

### Example 1

Nineteen female volunteers in their 30s to 50s with self-perceived sensitive skin, as identified by a preliminary questionnaire, were divided into two groups, and a lotion (P) containing γ-amino-β-hydroxybutyric acid having the composition shown in Table 1 and a placebo lotion (Q) were used by the respective groups for six weeks. Before the start of the test and after the completion of the test, the degree of the "prickling or tingling sensation" felt by the skin in daily life was evaluated using the following five-point scale, and the average values of the two groups were compared. It should be noted that the test was conducted under double-blind conditions. The results (the scores of each subject and the average values thereof) are shown in Table 2.

In Table 1, "PCA-Na 50% aq." means a 50% by mass aqueous solution of sodium pyrrolidone carboxylate.

### (Evaluation Criteria)

1: No prickling or tingling sensation is felt at all.
2: Slight prickling or tingling sensation is felt but to a negligible extent.
3: Prickling or tingling sensation is felt to an extent that causes a mild discomfort.
4: Prickling or tingling sensation is felt strongly and is uncomfortable.
5: Prickling or tingling sensation is felt very strongly and to an intolerable extent.

**[Table 1]**

| <Formula of Lotion> | | |
|---|---|---|
| Composition | P | Q |
| γ-amino-β-hydroxybutyric acid | 0.5 | - |
| water | 87.3 | 87.8 |
| Glycerin | 7 | 7 |
| Diglycerin | 3 | 3 |
| Citric acid | 0.03 | 0.03 |
| Trisodium citrate | 0.07 | 0.07 |
| Sodium benzoate | 0.1 | 0.1 |
| PCA-Na 50% aq. | 2 | 2 |
| pH | 5.37 | 5.50 |

**[Table 2]**

| <Results> | | | | | |
|---|---|---|---|---|---|
| ID | P group | | ID | Q group | |
| | Before the start of the test | After six weeks | | Before the start of the test | After six weeks |
| A | 3 | 1 | B | 4 | 3 |
| C | 3 | 2 | D | 3 | 3 |
| E | 4 | 1 | F | 3 | 4 |
| G | 4 | 2 | H | 4 | 4 |
| I | 3 | 3 | J | 4 | 3 |
| K | 3 | 2 | L | 4 | 3 |
| M | 4 | 2 | N | 3 | 4 |
| O | 2 | 1 | P | 2 | 2 |
| Q | 4 | 2 | R | 4 | 3 |
| | | | S | 4 | 3 |
| Average score value | 3.3 | 1.8 | Average score value | 3.4 | 3.2 |

From the results shown in Table 2, it was confirmed that in the group with the use of the lotion P containing γ-amino-β-hydroxybutyric acid, the average score of 3.3 before the start of the use decreased to 1.8 after the six weeks of the continuous use, indicating that the skin was improved to a state in which the "prickling or tingling sensation" was less likely to be felt. In contrast, no tendency toward improvement was observed in the group with the use of the placebo lotion Q.

### Example 2

Nineteen female volunteers in their 30s to 50s with self-perceived sensitive skin, as identified by a preliminary questionnaire, were divided into two groups, and a lotion (P) containing γ-amino-β-hydroxybutyric acid having the composition shown in Table 1 above and a placebo lotion (Q) were used by the respective groups for six weeks. The degree of the hypersensitivity was evaluated by measuring the electrical stimulation threshold (CPT value) at 250 Hz using a neurometer (manufactured by Neutron Co.). The measurements were performed before the start of the continuous use of the lotion, after four weeks of the continuous use, and after six weeks of the continuous use. The test was conducted under double-blind conditions. The results are shown in Fig. 1.

It should be noted that a larger electrical stimulation threshold (CPT value) indicates lower sensitivity to stimulation. In healthy skin, the value is approximately 15 (µA).

From the results shown in Fig. 1, it was confirmed that in the group with the use of the lotion P, the CPT value after six weeks of the continuous use was significantly increased as compared with that at the start of the test, indicating that the subjects became less sensitive to stimulation.

### Formulation Example 1 (Lotion)

### (Ingredients)

| | | |
|---|---|---|
| 1. | γ-amino-β-hydroxybutyric acid: | 1.0 (% by mass) |
| 2. | Citric acid: | appropriate amount (from 0.01 to 0.2) |
| 3. | Succinic acid: | appropriate amount (from 0.01 to 0.2) |
| 4. | Trisodium citrate: | appropriate amount (from 0.01 to 0.2) |
| 5. | Glycerin: | 5.0 |
| 6. | Diglycerin: | 2.0 |
| 7. | Sodium hyaluronate: | 0.1 |
| 8. | Carrageenan: | 0.15 |
| 9. | Ectoine: | 0.5 |
| 10. | N-methyl-L-serine: | 0.1 |
| 11. | Sodium benzoate: | 0.2 |
| 12. | Water: | balance |
| Total: | 100 | |

### (Preparation Method)

The ingredients 1 to 11 are sequentially added to water while stirring to dissolve, followed by filtration to prepare the lotion (with a pH value of from 4 to 7).

### Formulation Example 2 (Beauty Serum)

### (Ingredients)

| | | |
|---|---|---|
| 1. | γ-amino-β-hydroxybutyric acid: | 1.5 |
| 2. | Citric acid: | appropriate amount (from 0.01 to 0.2) |
| 3. | Trisodium citrate: | appropriate amount (from 0.01 to 0.2) |
| 4. | Glycerin: | 3.0 |
| 5. | Diglycerin: | 1.0 |
| 6. | 1,3-Butylene glycol: | 5.0 |
| 7. | Carboxyvinyl polymer: | (from 0.1 to appropriate amount 1.0) |
| 8. | Sodium hyaluronate: | 0.05 |
| 9. | Alanine: | 0.5 |
| 10. | Ectoine: | 0.05 |
| 11. | Arginine: | appropriate amount (from 0.1 to 1.0) |
| 12. | Phenoxyethanol: | 0.3 |
| 13. | Polyoxyethylene hydrogenated castor oil (60 E.O.): | 0.2 |
| 14. | Fragrance: | appropriate amount |
| 15. | Water: | balance |
| Total: | 100 | |

### (Preparation Method)

The ingredients 1 to 12 are sequentially added to water while stirring to dissolve. Separately, the ingredients 13 and 14 are mixed and then added to the previously prepared aqueous phase to prepare a beauty serum (with a pH value of from 5 to 7).

The blended amount of the ingredient 7 is adjusted so that the final viscosity measured at 30°C using a B-type viscometer is from 1,000 to 20,000 mPa·s.

### Formulation Example 3 (Emulsion)

### (Ingredients)

| | | |
|---|---|---|
| 1. | γ-amino-β-hydroxybutyric acid: | 0.2 (% by mass) |
| 2. | Citric acid: | appropriate amount (from 0.01 to 0.2) |
| 3. | Trisodium citrate: | appropriate amount (from 0.01 to 0.2) |
| 4. | Glycerin: | 5.0 |
| 5. | Carrageenan: | 0.2 |
| 6. | N-methyl-L-serine: | 0.2 |
| 7. | Water: | balance |
| 8. | Hydrogenated soybean phospholipid: | 0.5 |
| 9. | Cholesterol (phytosterol): | 0.5 |
| 10. | Sugar squalane: | 8.0 |
| 11. | Polyglyceryl-10 diisostearate: | 1.0 |
| 12. | Octyldodecyl myristate: | 3.0 |
| 13. | Isostearic acid: | 0.5 |
| 14. | Palmitic acid: | 0.1 |
| 15. | Dipropylene glycol: | 5.0 |
| 16. | 1,3-Butylene glycol: | 3.0 |
| 17. | Fragrance: | appropriate amount (from 0 to 0.5) |
| 18. | Phenoxyethanol: | 0.5 |
| 19. | Carboxyvinyl polymer: | appropriate amount (from 0.1 to 1.0) |
| 20. | Potassium hydroxide: | appropriate amount |
| Total: | 100 | |

### (Preparation Method)

The ingredients 1 to 7 are stirred and dissolved at 80°C, and gradually added to the ingredients 8 to 17 which were previously dissolved at 80°C, followed by dispersion and emulsification using a homomixer (HM), and then cooling. At around 50°C, the ingredients 18 to 20 are sequentially added and dispersed again using the HM, followed by cooling to 30°C to prepare the emulsion (with a pH value of from 5 to 7).

The blended amount of the ingredient 19 is adjusted so that the final viscosity measured at 30°C using a B-type viscometer is from 1,500 to 15,000 mPa·s.

### Formulation Example 4 (Cream)

### (Ingredients)

| | | |
|---|---|---|
| 1. | γ-amino-β-hydroxybutyric acid: | 2.0 (% by mass) |
| 2. | Citric acid: | appropriate amount (from 0.01 to 0.2) |
| 3. | Trisodium citrate: | appropriate amount (from 0.01 to 0.2) |
| 4. | Phytic acid: | 0.1 |
| 5. | Sodium benzoate: | 0.2 |
| 6. | Dipotassium glycyrrhizate: | 0.1 |
| 7. | Glycerin: | 5.0 |
| 8. | Maltitol solution: | 8.0 |
| 9. | Sodium hyaluronate: | 0.1 |
| 10. | PCA-Na: | 1.0 |
| 11. | Water: | balance |
| 12. | Hydrogenated soybean phospholipid: | 1.0 |
| 13. | Cholesterol (phytosterol): | 0.5 |
| 14. | Palmitic acid: | 0.2 |
| 15. | Behenyl alcohol: | 1.0 |
| 16. | Vegetable squalane: | 10.0 |
| 17. | Isostearic acid: | 0.5 |
| 18. | Cetanol: | 0.5 |
| 19. | Macadamia nut oil: | 3.0 |
| 20. | Jojoba oil: | 3.0 |
| 21. | Dipropylene glycol: | 5.0 |
| 22. | 1,3-Butylene glycol: | 3.0 |
| 23. | Fragrance: | appropriate amount (from 0 to 0.5) |
| 24. | Carboxyvinyl polymer: | appropriate amount (from 0.1 to 1.0) |
| 25. | Potassium hydroxide: | appropriate amount |
| Total: | 100 | |

### (Preparation Method)

The ingredients 1 to 11 are stirred and dissolved at 80°C, and gradually added to the ingredients 12 to 23 which were previously dissolved at 80°C, followed by dispersion and emulsification using a homomixer (HM), and then cooling. At around 50°C, the ingredients 24 and 25 are sequentially added and dispersed again using the HM, followed by cooling to 30°C to prepare the cream (with a pH value of from 5 to 7).

The blended amount of the ingredient 24 is adjusted so that the final viscosity measured at 30°C using a B-type viscometer is from 10,000 to 100,000 mPa·s.

Any of the agents for alleviating hypersensitivity according to the Formulation Examples 1 to 4 is capable of alleviating or relieving the degree of the hypersensitivity for the sensitive skin of hypersensitivity type with an apparently healthy appearance.

## Claims

1. An agent for alleviating skin hypersensitivity, comprising γ-amino-β-hydroxybutyric acid (A) as an active ingredient.

2. The agent for alleviating the hypersensitivity according to Claim 1, wherein a content of the ingredient (A) is from 0.005 to 2% by mass.

3. The agent for alleviating the hypersensitivity according to Claim 1 or 2, further comprising a polyhydric alcohol (B).

4. Use of γ-amino-β-hydroxybutyric acid (A) as an agent for alleviating skin hypersensitivity.

5. A method of using γ-amino-β-hydroxybutyric acid (A) as an agent for alleviating skin hypersensitivity.
